(19) Europäisches Patentamt
European Patent Office
Office européen des brevets

(11) Veröffentlichungsnummer: **0 017 882 B1**

# (12) EUROPÄISCHE PATENTSCHRIFT

(45) Veröffentlichungstag der Patentschrift: **10.02.82**

(51) Int. Cl.³: **C 07 C 65/24**

(21) Anmeldenummer: **80101843.3**

(22) Anmeldetag: **05.04.80**

## (54) Verfahren zur Herstellung von 3-Phenoxy-4-fluor-benzoesäure.

(30) Priorität: **19.04.79 DE 2915738**

(43) Veröffentlichungstag der Anmeldung: **29.10.80 Patentblatt 80/22**

(45) Bekanntmachung des Hinweises auf die Patenterteilung: **10.02.82 Patentblatt 82/6**

(84) Benannte Vertragsstaaten: **BE CH DE FR GB IT LI NL**

(56) Entgegenhaltungen:
**DE-C-150 323**
**DE-C-158 998**
**GB-A-23 974-1 904**
**GB-A-1 543 965**
**US-A-3 652 665**

(73) Patentinhaber: **BAYER AG, Zentralbereich Patente, Marken und Lizenzen, D-5090 Leverkusen 1, Bayerwerk (DE)**

(72) Erfinder: **Cölln, Reimer, Dr., In den Birken 67, D-5600 Wuppertal 1 (DE)**
Erfinder: **Priesnitz, Uwe, Dr., Heinrich-Heine-Strasse 42, D-4750 Unna-Massen (DE)**
Erfinder: **Klauke, Erich, Dr., Eichendorffweg 8, D-5063 Odenthal-Hahnenberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# Verfahren zur Herstellung von 3-Phenoxy-4-fluor-benzoesäure

Die Erfindung betrifft ein neues Verfahren zur Herstellung von 3-Phenoxy-4-fluor-benzoesäure.

Es sind bereits verschiedene Reaktionen von Halogen-fluor-benzolderivaten mit Phenolaten bekannt geworden, bei denen Fluor eher als ein anderes Halogen substituiert wird; der umgekehrte Fall ist noch nicht bekannt. So entsteht beispielsweise aus 4-Fluor-chlorbenzol und Kaliumphenolat als Hauptprodukt 4-Chlor-diphenylether (vergleiche DE-OS 2 619 489).

Die Reaktion von Pentafluor-chlorbenzol mit Natrium-pentafluorphenolat führt zu einem Isomerengemisch aus 4-Chlor-nonafluor-diphenylether und 2-Chlor-nonafluor-diphenylether (vergleiche US-PS 3 637 866).

2,5-Dichlor-4-fluor-3-nitro-benzoesäure und Phenol reagieren in wässriger Natriumhydrogencarbonatlösung unter Bildung von 2,5-Dichlor-3-nitro-4-phenoxy-benzoesäure als Hauptprodukt (vergleiche J.Med.Chem. 15 (1972) 79–83).

Weiter ist bekannt, dass die Bildung von Diphenyläthern aus Halogen-benzolen und Phenolaten, beispielsweise die Herstellung von 3-Phenoxy-toluol aus Brombenzol und Kalium-m-cresolat, mit Kupferbronze katalysiert werden kann (vergleiche GB-PS 1 052 390 und DE-OS 2 619 489).

Ferner ist bekannt, dass Diphenyläther aus Halogenbenzolen und Phenolaten vorteilhaft in Gegenwart von Kaliumsalzen hergestellt werden können (vergleiche DE-OS 2 242 519).

Es wurde gefunden, dass man 3-Phenoxy-4-fluor-benzoesäure der Formel I

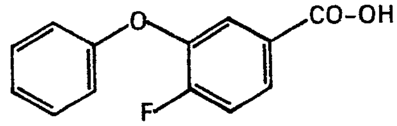

(I)

bzw. deren Alkalisalze erhält, wenn man 3-Brom-4-fluor-benzoesäure der Formel II

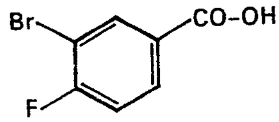

(II)

bzw. deren Alkalisalze mit Kaliumphenolat oder mit Natriumphenolat in Gegenwart eines Kaliumsalzes, jeweils in Gegenwart von Kupfer oder einer katalytisch wirksamen Kupferverbindung und unter Verwendung von Phenol als Verdünnungsmittel bei Temperaturen zwischen 100 und 250°C umsetzt.

Es ist ausgesprochen überraschend zu bezeichnen, dass nach dem erfindungsgemässen Verfahren praktisch nur 3-Phenoxy-4-fluor-benzoesäure aus 3-Brom-4-fluor-benzoesäure und Phenolat entsteht, da nach dem Stand der Technik eher eine Substitution des Fluors unter Bildung von 3-Brom-4-phenoxy-benzoesäure zu erwarten war.

Die erfindungsgemässe Reaktion kann, wenn das Kaliumsalz der 3-Brom-4-fluorbenzoesäure sowie Kaliumphenolat eingesetzt werden, durch folgendes Formelschema skizziert werden:

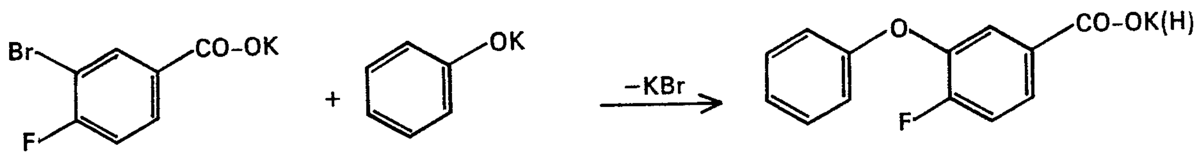


Die als Ausgangsverbindung einzusetzende 3-Brom-4-fluor-benzoesäure ist bereits bekannt (vergleiche Canad. J. Chem. 38 (1960), 2441–2449).

Man erhält sie auf technisch-einfache Weise, wenn man 4-Chlor-benzoylchlorid durch Umsetzung mit Kaliumfluorid in 4-Fluor-benzoylfluorid umwandelt, letzteres zu 3-Brom-4-fluor-benzoylfluorid bromiert und anschliessend eine alkalische Hydrolyse durchführt. Dieses Verfahren wurde bisher noch nicht beschrieben. Es lässt sich durch folgendes Formelschema wiedergeben:

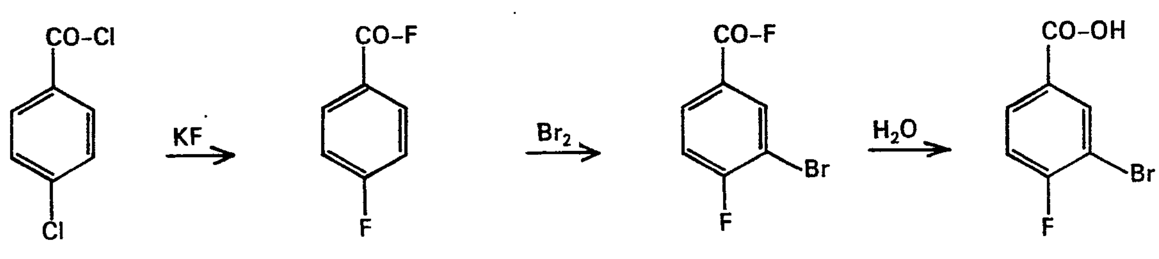


Im Einzelnen wird zunächst 4-Chlor-benzoylchlorid mit Kaliumfluorid in Tetramethylensulfon bei Temperaturen zwischen 200 und 220°C umgesetzt und das Reaktionsgemisch destillativ aufgearbeitet. Man erhält 4-Fluor-benzoylfluorid vom Siedepunkt 53°C/20 mBar (Brechungsindex: $n_D^{20}$ = 1,4792). In einer zweiten Stufe wird 4-Fluor-benzoylfluorid in Gegenwart von 1% Eisen (III)-chlorid bei 70 bis 75°C mit elementarem Brom bromiert. Bei einem Ansatz von 1 Mol erhält man

nach Destillation 40 g unverändertes Ausgangsmaterial und 182 g eines Gemisches aus 3-Brom-4-fluor-benzoylfluorid (Siedepunkt 82–83°C/15 mBar; Brechungsindex: $n_D^{20}$ = 1,5315; Schmelzpunkt 32–34°C) und 3-Brom-4-fluor-benzoylbromid (Siedepunkt 123°C/15 mBar; Schmelzpunkt 35–37°C). Durch alkalische Hydrolyse des Gemisches aus Säurefluorid und Säurebromid erhält man praktisch quantitativ die Säure (II) vom Schmelzpunkt 136–137°C. Das erfindungsgemässe Verfahren zur Herstellung von 3-Phenoxy-4-fluor-benzoesäure (I) wird unter Verwendung von überschüssigem Phenol als Verdünnungsmittel durchgeführt.

Als Katalysatoren werden Kupfer oder Kupferverbindungen verschiedener Oxidationsstufen verwendet.

Beispielsweise seien Kupfer, Kupfer(I)oxid, Kupfer(II)oxid, Kupfer(I)chlorid, Kupfer(II)chlorid, Kupfer(I)bromid, Kupfer(I)iodid und Kupfersulfat genannt.

Die Reaktionstemperatur liegt beim erfindungsgemässen Verfahren zwischen 100 und 250°C, vorzugsweise zwischen 150 und 200°C. Die Umsetzung wird im allgemeinen bei Normaldruck durchgeführt.

Auf 1 Mol 3-Brom-4-fluor-benzoesäure setzt man zwischen 1,5 und 5 Mol, vorzugsweise zwischen 2 und 3 Mol Phenolat ein; dazu kommen noch 3 bis 10 Mol Phenol als Verdünnungsmittel.

An Katalysator werden zwischen 0,001 und 0,1 Mol, vorzugsweise zwischen 0,005 und 0,05 Mol je Mol 3-Brom-4-fluor-benzoesäure eingesetzt.

Die Mischung aus den Ausgangsstoffen, dem Katalysator und dem Verdünnungsmittel wird bei Raumtemperatur hergestellt, unter Rühren auf die erforderliche Reaktionstemperatur erhitzt und ungefähr eine Stunde lang bei dieser Temperatur gehalten.

Zur Aufarbeitung lässt man das Reaktionsgemisch auf etwa 100°C abkühlen, verdünnt mit Toluol und verdünnter Salzsäure, trennt nach Filtration die organische Phase ab, trocknet, filtriert und destilliert Toluol sowie Phenol weitgehend ab. Aus dem Rückstand wird der Rest des Phenols durch Wasserdampfdestillation entfernt.

Das Rohprodukt erhält man aus der dabei entstehenden wässrigen Dispersion nach Abkühlen in fester Form. Als Reinheitskriterium dient – nach Überführen in den Methylester – das Gaschromatogramm.

Die nach dem erfindungsgemässen Verfahren herzustellende 3-Phenoxy-4-fluor-benzoesäure (I) kann als Zwischenprodukt zur Herstellung von insektizid und akarizid wirksamen Pyrethroiden verwendet werden (vergleiche DE-OS 2 709 264). So erhält man daraus beispielsweise den als Zwischenprodukt für Pyrethroide bekannten 3-Phenoxy-4-fluor-benzylalkohol durch Umsetzung mit einem starken Reduktionsmittel, wie z.B. Lithiumtetrahydridoaluminat (Lithiumalanat), bei Temperaturen zwischen 0 und 100°C, gegebenenfalls unter Verwendung eines Verdünnungsmittels, wie z.B. Tetrahydrofuran.

Beispiel 1

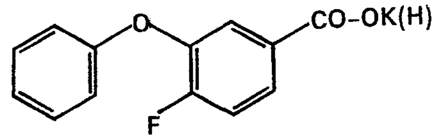

Man mischt 109,5 g (0,5 Mol) 3-Brom-4-fluor-benzoesäure, 250 g Phenol, 146 g (1,1 Mol) trockenes Kaliumphenolat und 1,0 g Kupfer(II)oxid in einem 2 l-Rührkolben und erhitzt die Mischung unter Rühren bis zum Sieden. Die Innentemperatur beträgt hierbei ca. 187°C. Nach einer Stunde Reaktionsdauer lässt man abkühlen. Solange die Mischung noch nicht erstarrt ist, also bei etwa 100°C, setzt man 500 ml Wasser, 500 ml Toluol und 80 g konzentrierter Salzsäure hinzu, saugt nach Abkühlung von wenig Ungelöstem ab und trennt die Phasen. Die organische Phase wird getrocknet und im Vakuum zunächst vom Toluol und dann vom Hauptteil des Phenols befreit. Der Rest des Phenols wird durch Wasserdampf übergetrieben. Die sich bildende wässrige Anschlämmung lässt man unter starkem Rühren erkalten, wobei das Reaktionsprodukt in fester absaugbarer Form anfällt. Nach dem Trocknen erhält man 110,4 g eines bräunlichen bröckligen Festproduktes.

Nach der gaschromatographischen Analyse (durchgeführt nach Überführung der Säuren in ihre Methylester) besteht dieses aus
3% 3,4-Diphenoxy-benzoesäure
64% 3-Phenoxy-4-fluor-benzoesäure
19% 4-Fluorbenzoesäure
und einigen geringeren Verunreinigungen.
Das Hauptprodukt wird demzufolge bei dieser Reaktion mit einer Ausbeute von 60,9% der Theorie gebildet. Die als Nebenprodukt entstandene 4-Fluorbenzoesäure wird zu 30% der Theorie gebildet und kann durch direkte Bromierung in das Ausgangsprodukt zurücküberführt werden. Stellt man diese Möglichkeit in Rechnung, erhöht sich die obige Ausbeute (jetzt bezogen auf den Umsatz:) auf 87%.

Die nach üblichen Methoden isolierte reine 3-Phenoxy-4-fluor-benzoesäure besitzt einen Schmelzpunkt von 145°C.

**Patentanspruch**

1) Verfahren zur Herstellung von 3-Phenoxy-4-fluor-benzoesäure der Formel I

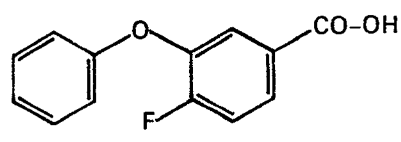

(I)

bzw. deren Alkalisalzen,
dadurch gekennzeichnet, dass man 3-brom-4-fluor-benzoesäure der Formel II

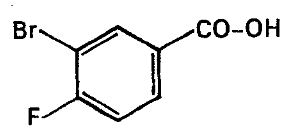

(II)

bzw. deren Alkalisalze
mit Kaliumphenolat oder mit Natriumphenolat in Gegenwart eines Kaliumsalzes, jeweils in Gegenwart von Kupfer oder einer katalytisch wirksamen Kupferverbindung und unter Verwendung von Phenol als Verdünnungsmittel bei Temperaturen zwischen 100 und 250°C umsetzt.

**Claim**

1) Process for the preparation of 3-phenoxy-4-fluoro-benzoic acid of the formula I

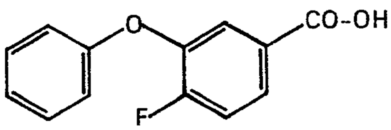

(I)

or an alkali metal salt thereof, characterised in that 3-bromo-4-fluoro-benzoic acid of the formula II

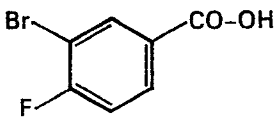

(II)

or an alkali metal salt thereof, is reacted with potassium phenolate or with sodium phenolate in the presence of a potassium salt, in each case in the presence of copper or a copper compound having a catalytic action, and using phenol as the diluent, at temperatures between 100 and 250°C.

**Revendication**

1) Procédé de préparation de l'acide 3-phénoxy-4-fluoro-benzoïque de formule I

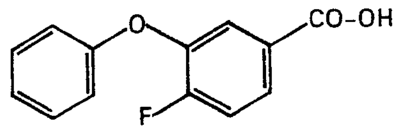

(I)

et de ses sels alcalins,
caractérisé en ce que l'on fait réagir l'acide 3-bromo-4-fluoro-benzoïque de formule II

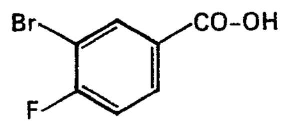

(II)

ou ses sels alcalins respectivement,
avec le phénate de potassium ou avec le phénate de sodium, en présence d'un sel de potassium, dans tous les cas en présence de cuivre ou d'un composé de cuivre possédant une activité catalytique et en utilisant le phénol comme diluant à des températures de 100 à 250°C.